# EUROPEAN PATENT SPECIFICATION

(11) **EP 4 338 773 B1**
(45) Date of publication and mention of the grant of the patent: **02.04.2025**
(21) Application number: 22195704.6
(22) Date of filing: 14.09.2022
(51) Int. Cl.: A61M 11/00, A61M 15/00

(54) **A NEBULIZATION SYSTEM WITH A DRUG SUPPLY UNIT FOR AN INHALATION DEVICE**
VERNEBELUNGSSYSTEM MIT EINER ARZNEIMITTELZUFÜHREINHEIT FÜR EINE INHALATIONSVORRICHTUNG
SYSTÈME DE NÉBULISATION AVEC UNE UNITÉ D'ALIMENTATION EN MÉDICAMENT POUR UN DISPOSITIF D'INHALATION

(43) Date of publication of application: 20.03.2024
(73) Proprietor: Activoris Medizintechnik GmbH, 35285 Gemünden (Wohra) (DE)
(72) Inventor: Dunne, Stephen T., Porto 4150-044 (PT); Fischer, Axel, 34630 Gilserberg (DE); Kolb, Tobias, 82061 Neuried (DE)
(74) Representative: Tergau & Walkenhorst Intellectual Property GmbH

(56) References cited:
- WO-A1-2020/097085
- WO-A1-2021/069972
- WO-A1-2022/101851
- US-A- 6 145 703
- US-A1- 2007 076 067
- US-A1- 2015 352 301
- US-A1- 2019 388 627

## Description

The invention relates to a medical inhalation device, in particular for nebulizing a drug and delivering its active ingredient to the respiratory system of a patient during treatment of the patient, using a drug supply unit in which the drug formulation solution is provided in a cartridge, in particular a pre-filled cartridge. It further relates to a drug supply unit for such inhalation device, and to a nebulization system comprising such drug supply unit.

A variety of drugs is intended to be delivered to the patient via the respiratory system, by the way of inhalation. Such inhalation may be effected in two different types, one in the form of so-called dry inhalation and the other in the form of so-called moist inhalation or liquid inhalation.

In the type of dry inhalation, the active ingredient is inhaled as a powder-air mixture, and the device used for such dry inhalation typically is referred to as inhaler. The advantage of such dry inhalation is that the administered dose is always precisely determined. The disadvantage of the inhalers, however, is that for correct application it is necessary to coordinate the inhalation exactly with the release of the stroke. Therefore, inhalers are less suitable for babies and small children. Dry inhalation is most often used to administer bronchodilators and anti-inflammatory agents. Emergency medications for asthma and other chronic lower respiratory diseases are also carried in inhalers.

For moist inhalation or liquid inhalation, in contrast, typically so-called inhalation devices, also referred to as nebulizers, are used. In this type of inhalation, an aerosol from the active substance is generated, which then is provided to the patient for aspiration. The aerosol to be inhaled can be produced by different techniques. Depending on the technology, a distinction is made between ultrasonic, compressed air (or compressor) or membrane nebulizers. The active substance in such systems is inhaled in tiny droplets. The size of the droplets can be precisely determined, thus optimally reaching either the upper, middle, or lower respiratory tract. Therefore, the inhalation devices are suitable for a wide range of respiratory diseases.

An advantage of such nebulizers is that they do not require any special breathing technique since inhalation can also be performed through a mask. Therefore, inhalation devices are particularly suitable for small children and babies. The disadvantage of this method is the longer time needed to administer the same amount of active ingredient.

For the application of medical substances or drugs via nebulizers, the liquid containing the active ingredient may be supplied to an integrated container of the nebulizer systems, from which it is delivered to the actual nebulizer unit, typically an ultrasonic or membrane nebulizer. In such systems, however, no sterile environment may be maintained, thereby limiting the potential field of applications. Further, handling of the components requires various steps and may be difficult for the individual, in particular if the patient himself is expected to use the system. In order to overcome these deficiencies, the drug may be delivered to the nebulizer in pre-filled cartridges. The use of such drug containing cartridges in soft mist inhaler systems is known, e. g., from WO 2016/012102 A1. Such a soft mist inhaler, however, is not strictly a nebulizer, and it is a multi dose device with very small doses at 15 ml so that a cartridge may contain up to 180 doses.

Cartridges are commonly used to store drug formulations. They are described in ISO 13926-1. The cartridge comprises of a glass or plastic barrel, a pierceable septum held by a crimped metal ferrule at one end and a stopper/piston at the other. Usually, an injection needle is manually attached by the user before use by screwing a double ended needle, comprising of an injection and a septum needle, on to a cartridge holder or other device such as a pen holding the cartridge. This perforates or pierces the septum making a fluid pathway between the cartridge drug contents and the injection needle. Such arrangements, in which the cartridge typically contains one large dose to be delivered to the patient, are typically used in pen injectors such as insulin pens and cartridge-based dentistry syringes.

An arrangement with a septum needle and a delivery nozzle instead of an injection needle may also be used to deliver to drug delivery device such as a nebulizer or inhale or other delivery device.

Cartridges are available in both ready to fill and much cheaper bulk formats. They can be filled like syringes by capping, filling and stoppering or bubble free by stoppering, filling and capping.

Some prior art nebulizers are also known from the documents US 6 145 703 A, WO 2021/069972 A1, WO 2020/097085 A1, US 2019/388627 A1, WO 2022/101851 A1, US 2007/076067 A1 and US 2015/352301 A1.

### SUMMARY OF THE INVENTION

The present invention provides a nebulization system according to claim 1, and an inhalation device according to claim 4. Further preferred embodiments of the present invention are defined in the dependent claims.

### SUMMARY OF THE DISCLOSURE

Reference will be made in the following description to various examples or aspects of the present technology. Examples or aspects disclosed herein which do not fall under the scope of the appended claims do not form part of the present invention, but are useful for understanding the principles of the invention. The scope of the present invention is defined by the appended claims.

The present technology relates to an inhalation device, in particluar a nebulizer, including a cartridge-based assembly designed to deliver a drug formulation to the nebulizer, which is particularly suitable for delivering a medical drug to a patient. It is an object of the present technology to provide a drug supply unit for such an inhalation device, based upon the use of pre-filled cartridges, that is particularly user friendly and ease and safe to use even for self-application of the drugs.

According to the present technology, this object is achieved with a drug supply unit for an inhalation device comprising a cartridge containing a drug formulation solution, with a housing, in which the cartridge is arranged so as to be displaceable in a longitudinal direction, the housing having a septum needle in an end region, which, in an active position of the cartridge, pierces a septum arranged at the distal end of the cartridge when the cartridge is completely displaced in the longitudinal direction towards the end region, and having a releasable lock which fixes the cartridge in a passive position within the housing in such a way that the septum needle is held at a distance from the septum.

The technology is based upon the consideration that for a safe, easy-to-use design of an inhalation device, the intended use of pre-filled cartridges for supplying the drugs or active substances may be very convenient. Such pre-filled cartridges, comparably to the use of pre-filled syringes, may be delivered to the user in sealed conditions. Access to the drug or active substance within the cartridge may then be provided by using a septum needle that pierces the septum at one end of the cartridge which is typically provided as sealing element for such cartridges. For easy and reliable use, engaging of the cartridge with the septum needle should be designed carefully and reliable, and for this purpose the technology provides a housing in which the cartridge is allowed to slide along a longitudinal direction. At one end region, the septum needle is provided, and for accessing the interior of the cartridge, the cartridge may be moved within the housing towards the septum needle until the septum needle pierces the septum and thus accesses the interior. This "opening" step of the sealed cartridge may be performed and highly controlled and stable conditions and is therefore easy to perform even by an untrained user.

In this setup, an unintended opening or access to the interior of the container should reliably be avoided. For this purpose, the technology provides a mechanism that keeps the cartridge out of range of the septum needle until opening purposefully is initiated by the user. For this purpose, the technology provides a locking mechanism that keeps the cartridge in a first, passive position far enough away from the septum needle. The cartridge may then be moved into an active position, in which the septum needle is allowed to penetrate the septum, after intentional disengaging or releasing of the lock by the user.

In a preferred embodiment, the lock of the drug supply comprises a locking pin which in a locking position protrudes into the interior of the housing, thereby mechanically blocking the movement of the cartridge within the housing. In unlocked mode, in which the cartridge should be allowed to move within the housing, the locking pin preferably is then allowed to move radially outward from the interior of the housing.

In one aspect of the technology, the drug supply system is part of a nebulization system. The nebulization system, in addition to the drug supply unit as described above, in this aspect of the technology further comprises a nebulizer body with a nebulization chamber, wherein said septum needle is in fluid communication with a nozzle extending into said nebulization chamber. Accordingly, when the catridge within the housing is brought into active position, the interior of the cartridge, via septum needle and nozzle, is fluidly connected with the nebulization chamber, and thus the drug formula solution is allowed to flow from the cartridge into the nebulization chamber. In an aspect of the technology, unlocking of said lock is then effected by a twisting or rotating motion of the housing around its longitudinal direction relative to the nebulizer body.

In another aspect of the technology, the inhalation device comprising a nebulization system of the type presented above is connected to a power supply and control unit by an electric connection system, wherein said electric connection system comprises a number of paired connector elements that are individualized with respect to the drug to be delivered. In other words: "paired connector elements" is supposed to mean that, by means of geometry, design, or electronic functions, only paired pieces of elements may be connected to each other. According to an aspect of the technology, this pairing is based upon the elements being associated to individual drugs or dosages. In this way, a safety feature can be provided that allows coupling of the elements, i. e. coupling the nebulization system to the electric power and control unit, only under the condition that both parts refer to the same intended drug and/or dosage. Therefore, if a patient is reusing the power and control unit, but using one-time nebulizers, only components providing the correct medication for the individual patient can be connected to the patient's multi-use power and control unit.

In view of the high requirements with respect to reliability and precision in the context of drug delivery, the nebulizer according in a preferred embodiment comprises a nebulizing element making use of a vibrating mesh for atomizing the delivered liquid.

According to various aspects of the technology, one or more of the following features are provided, in particular in any suitable combination:
- an atomizer comprising a mesh that, when vibrating, nebulizes a supplied liquid;
- an outer casing or housing or syringe housing holding,
- a cartridge having a glass or plastic barrel or generally tubular barrel, a needle pierceable septum or sealing element held by a crimped metal ferrule disposed at the distal end of the barrel and a stopper/piston disposed at the other end of the barrel to contain the medicament or other formulation between the stopper/piston member and the septum or sealing element,
- a plunger rod in communication with the cartridge stopper/piston,
- a needle arrangement comprising of a deliver nozzle at one end and a septum needle at the other in fluid communication with one another,
- where the needle arrangement is rigidly held in the outer casing,
- where the outer casing or syringe is placed into a nebulizer,
- where the filled cartridge is held in the outer casing in a first position,
- where the user can push the cartridge relative and towards to the septum needle into a second position where the septum needle perforates the cartridge septum making the syringe ready for use.

The cartridge may be held in the first position by interference fit or friction between the cartridge barrel and the outer casing or housing.

The cartridge may be held in the first position by a deformable or movable stop on the cartridge shoulder or cap.

The cartridge may be further held in the first position by a lock on the cartridge shoulder or cap that is released by activating a switch or button or by rotating the syringe relative to the nebulizer or any other means.

The cartridge may be held in the first position by a combination of two or all three of the above methods.

To use the device the user simply pushes the plunger rod with sufficient force to move the cartridge from the first position to the second position to overcome the interference fit or friction and or the deformable or movable stop. This moves the cartridge to the second position towards the needle arrangement perforating the septum making the system ready for delivering the drug to the inhaler. Further pushing of the plunger rod then delivers the drug formulation to the nebulizer.

The cartridge may be filled bubble free or with an air bubble. This makes no difference to the use of the device.

The device may have a locking mechanism that holds the cartridge in place before use. If the device has a locking mechanism this needs to be unlocked by a switch or button or other means so that the cartridge can be pushed to the second position by depressing the plunger rod.

The syringe or outer casing may be supplied separately from the nebulizer or pre-assembled with the nebulizer. If supplied separately the cartridge lock may independent from the nebulizer and the unlocking may take place when the syringe is pushed into the nebulizer and the syringe is rotated relative to the nebulizer. If supplied pre-assembled the unlocking may take place when the syringe is rotated relative to the nebulizer.

The device may include a nozzle cap to cover the nozzle prior to use. The nozzle cap can be used to lock the cartridge at the first position before use. This locking feature may be part of the cap or the housing in which case the lock is held in the locked position by the cap. The cap may have an opening that allows the nozzle to protrude through. When pushing the syringe into the nebulizer the cap may be pushed back freeing the lock. The lock is only finally released when the syringe is rotated relative to the nebulizer allowing the cartridge to move forward piercing the septum and delivering the drug contents into the nebulizer.

The whole assembly including the cartridge may be terminally sterilized in a blister or other outer packaging. Depending on the drug type and the packaging and device materials the drug filled system can be sterilized using moist heat (steam), dry heat, gamma radiation or other radiation, ethylene oxide gas, vaporized hydrogen peroxide, chlorine dioxide gas, vaporized peracetic acid and nitrogen dioxide.

The cartridge barrel may be made of glass or plastic such as COC or COP and in accordance with ISO 13926-1.

The current technology is a great improvement over the existing state of the art drug containers for nebulizers. The drug is stored in a cartridge with the drug only in contact with the barrel material, glass or plastic and an elastomeric material. This is the same as with storage in a vial. Additionally, the cartridge may have the superior baked-on silicone for improved stopper travel and drug stability and the whole system with filled cartridge may be terminally sterilized.

An embodiment of the technology is explained in more detail with reference to a drawing. Therein show:
- Fig. 1: a conventional standard ISO 13926 cartridge with a plunger rod in place,
- Fig. 2: a nebulization system comprising the cartridge of Fig. 1,
- Fig. 3a,b: two user steps for activating the nebulization system according to Fig. 2 for use,
- Fig. 4a,b: two user steps when preparing nebulization in the nebulization system of Fig. 2 by delivering drug formulation to nebulizer chamber,
- Fig. 5: an inhalation device ready for use,
- Fig. 6: another embodiment of a cartridge in housing,
- Fig. 7: a nebulization system comprising the cartridge of Fig. 6,
- Fig. 8a,b: two user steps when preparing nebulization in the nebulization system of Fig. 7 by delivering drug formulation to nebulizer chamber,
- Fig. 9a,b: an alternative embodiment of a nebulization system, and
- Fig. 10a,b: an alternative embodiment of a cartridge in a housing with another form of locking mechanism.

Identical parts are provided with the same reference signs in all figures. The technology will be explained below primarily with reference to the embodiments shown. However, other embodiments are of course also conceivable and covered by the present technology.

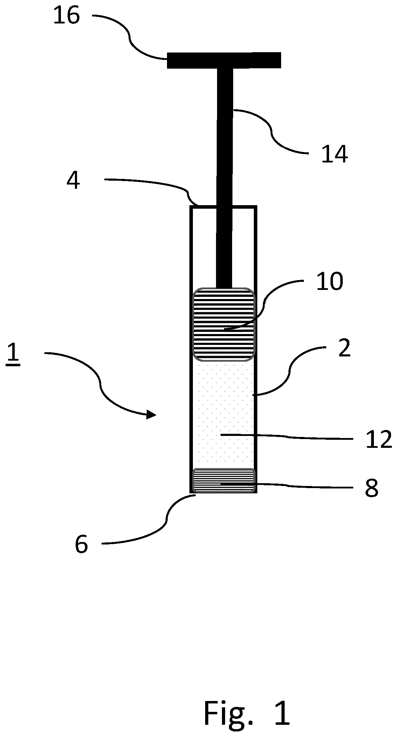

The cartridge 1 according to Fig. 1 has a plastic or glass barrel 2, providing the actual container for a liquid medication or drug. The barrel 2 has an open or proximal end 4 and a sealed or distal end 6 closed with a cap or septum 8. A movable stopper 10 inserted into the open end 4 and the septum 8 seal a drug formulation solution 12, in particular a liquid, within the barrel 2. A plunger rod 14 with finger pad 16 is engaged with stopper 10 such that stopper 10 can be pushed forwards and backwards in the barrel 2 by plunger rod 14.

Such cartridges 1 are fully described in ISO 13926-1 and can have glass or plastic barrels 2.

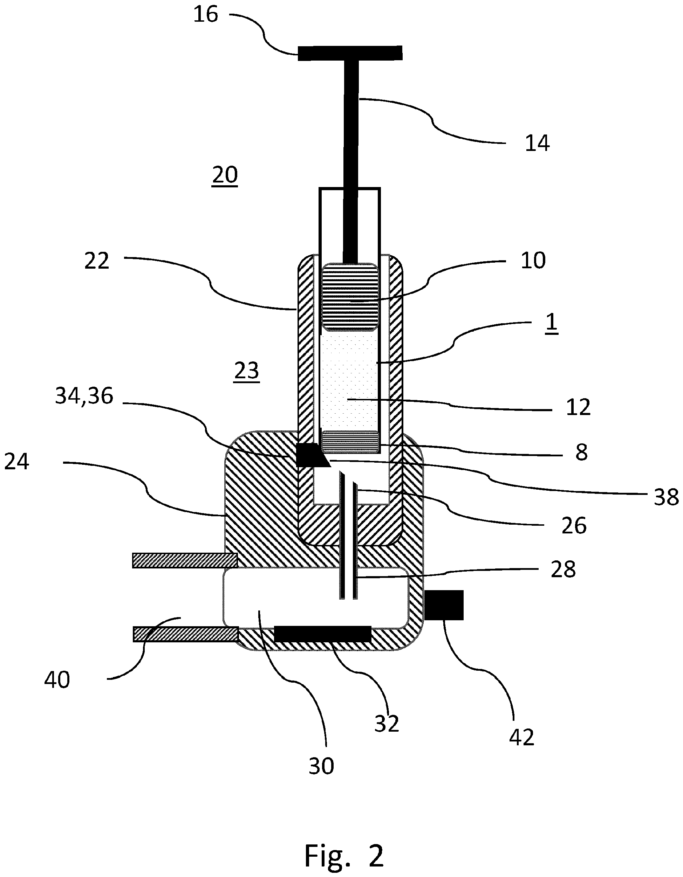

The nebulizer system 20 in accordance with the present technology is shown in Fig. 2. In its basic concept it is designed to be particularly useful and convenient for nebulizing and delivering the liquid drug formulation solution 12 contained in a cartridge 1 of the type shown in Fig. 1. The cartridge 1 is mounted within a casing or housing 22 at a first position. The assembly of cartridge 1 and housing 22 forms a drug supply unit 23. Housing 22 is mounted in a nebulizer body 24. Fixed to housing 22, a septum needle 26 is provided in fluid communication with a delivery nozzle 28. Nozzle 28 within the nebulizer body 24 extends into a nebulizer chamber 30 in which the actual nebulization element 32 is provided. In the embodiment shown, nebulization element 32 is an ultrasonic nebulizer. In accordance with an aspect of the technology and in a preferred embodiment, however, nebulization element 32 comprises a vibrating mesh.

In one independently inventive aspect of the embodiment shown, the ensemble of cartridge 1 in combination with plunger rod 14, housing 22, and septum needle 26 is functionally and in its design and layout comparable and equivalent to a prefilled and cartridge-loaded syringe, the main difference being that in the embodiment shown, the septum needle 26, rather than being in fluid communication with an injection needle, is in fluid communication with the nozzle 28.

In the status as shown in Fig. 2, cartridge 1 is locked in a given first position within housing 22 in order to prevent unintended, accidental movement. In order to prevent cartridge 1 from unintended axial moving under user force on plunger rod 14 in the direction towards the septum needle 26 to a second position within housing 22, a lock 34 is provided. Lock or locking pin 34 in the embodiment shown is designed as a locking wedge 36 with an angled side in the status shown in Fig. 2 protruding into the interior of housing 22 and with its distal end or angled side 38 engaging with distal end 6 of barrel 2. In its longitudinal direction, locking pin 36 in turn is blocked from moving outwards by the nebulizer body 24.

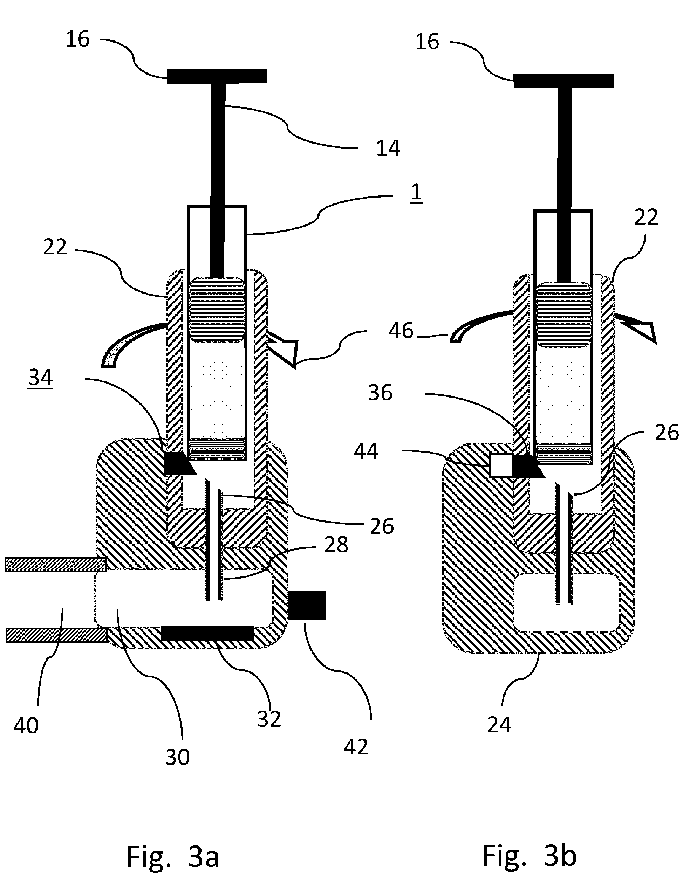

Nebulizer body 24 further is provided with a mouthpiece 40 for delivering the aerosolized drug formulation to the user. Further, an electrical connector 42 is provided at nebulizer body 24 that supplies the necessary power and control signals/nebulizing instructions to the nebulization element 32. In this embodiment the cartridge housing 22 and the nebulizer body 24 are supplied assembled to user. In an aspect of the technology, the total (preassembled) assembly may be terminally sterilized after assembly and supplied in a sterile blister to the user. Figs. 3 a,b show the first user steps required to operate the inhalation device 20. For operating the nebulization system 20, lock 34 must be disengaged in order to enable the user to move the cartridge 1 within the housing 22 towards the septum needle 26 such that it can penetrate septum 8 of cartridge 1. According to an aspect of the present technology, in the embodiment shown unlocking of lock 34 is effected by a twisting or rotating motion of housing 22 around its longitudinal direction relative to nebulizer body 24 until locking pin 36 rotationally overlaps with a corresponding recess 44 or cavity in nebulizer body 24. Recess 44 is designed such that locking pin 26 can slide freely therein such that in this orientational direction it is free to move radially outward and thereby no longer impedes cartridge 1 from being moved forward in housing 22 in the direction towards septum needle 26.

For illustration, Fig. 3a shows the assembly in the first position as shown in Figure 2. Figure 3b, in turn, shows the assembly after the user has rotated or twisted the cartridge housing 22 relative to nebulizer body 24 as shown by arrow 46. (Accordingly, in Fig. 3b and subsequent Figs. the nebulizer body 24 is shown in a rotated position realive to the cartridge housing 22). By rotating housing 22 relative to nebulizer body 24 the lock 34 therefore is free to move outwards thereby freeing cartridge 1 so it can be pushed by user towards septum needle 26.

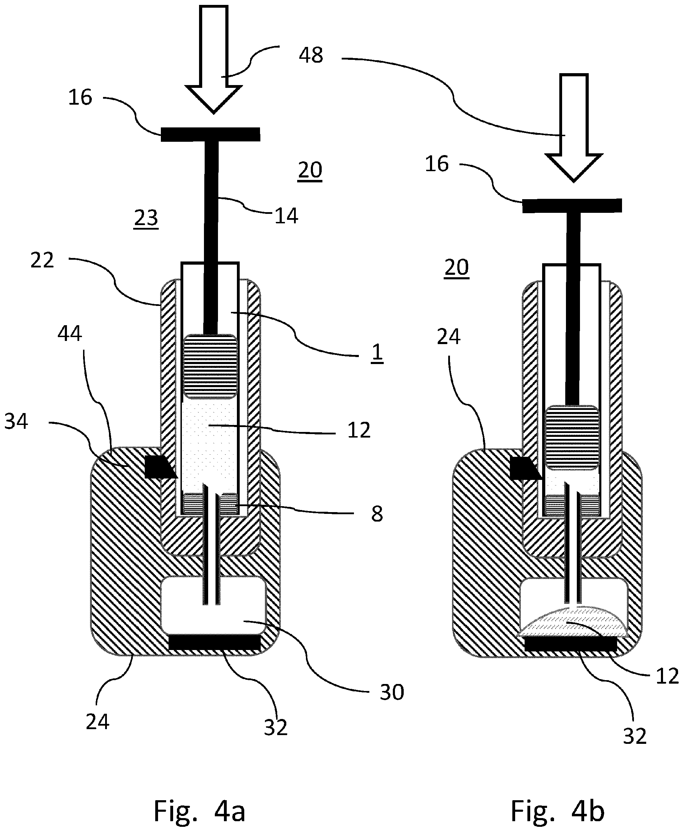

Fig. 4a shows the assembly after the user has pushed the cartridge 1 downwards towards the nebulizer chamber 30 as shown by arrow 48. As indicated in step according to Fig. 3b, lock 34 has been unlocked by forcing locking pin 36 sideways into cavity 44 in nebulizer body 24. As a consequence of pushing cartridge 1 downwards, septum needle 26 has perforated septum 8, thereby opening a fluid connection between the interior of barrel 2 and, via nozzle 28, nebulizer chamber 30. Therefore, in the state shown in Fig. 4a, drug formulation solution 12 now is able to flow from barrel 2 into nebulizer chamber 30. Further pushing of plunger 14 delivers the drug formulation contents 12 to the nebulizer chamber 30 as shown in Fig. 4b where the contents 12 are stored in a reservoir ready for atomization by nebulizer element 32.

In total, the drug supply unit 23 as described above comprises the cartridge 1 containing a drug formulation solution 12 and the housing 22, in which the cartridge 1 is arranged so as to be displaceable in a longitudinal direction. The housing 22 also has a septum needle 26 in an end region, which, in an active position of the cartridge 1, pierces the septum 8 arranged at the distal end 6 of the cartridge 1 when the cartridge 1 is completely displaced in the longitudinal direction towards the end region. The supply unit 23 also has a releasable lock 34 which fixes the cartridge 1 in a passive position within the housing 22 in such a way that the septum needle 26 is held at a distance from the septum 8 if the system is not ready for dispensing the drug formulation solution yet.

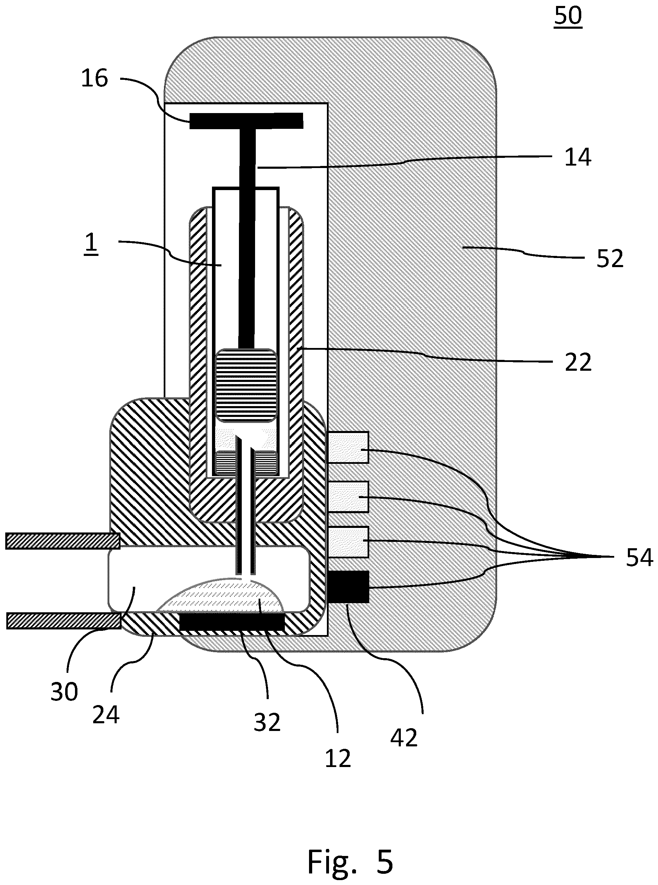

The completed assembly or nebulization system 20 in the status as shown in Fig. 4b can be converted into a fully operative inhalation device 50 by combining it with a corresponding power supply unit 52. This complete inhalation device 50 is shown in Fig. 5. More specifically, Fig. 5 shows the assembly mounted in power source and controlling unit 52. Controlling unit or power supply unit 52 determines the correct power and nebulizing time for each drug type and dose. Further, power and control unit 52 also provides an outer shell or casing for the assembled device, into which the nebulizer system 20 only can be inserted with rod 14 pushed into barrel 2, i. e. in "primed" or activated condition of nebulizer system 20 with the drug formulation solution 12 transferred into nebulization chamber 30.

According to an individually inventive aspect of the technology, the electric connection between electrical connector 42 and its counterpart in power supply unit 52 is individualized in the sense that their shape and geometry are individual and exclusive for an individual or specific drug. In other words, electrical connector 42 (and its matching counterpart in power supply unit 52) is unique to each drug and dose type and slots into the correct female connector 54 on controlling unit 52. With this inventive concept, the assembly of cartridge 1 and the other components altogether providing nebulization system 20 may be preassembled and provided to the user as a complete assembly, whereas the power supply unit 52 may be designed as a multiple use unit that may be reused by the user several times, Due to the drug specific, indivualized electrical connection system, the user will have an additional safety feature making sure that he is about to use the correct drug as intended: due to the drug-specific design of the connection, only nebulization systems 20 containing the correct drug in correct dosage can be connected to the handpiece or power and control unit 52.

In another aspect of the technology, as an additional safety feature, for actual nebulization, plunger 14 must be fully depressed before the nebulization system 20 can be slotted into place and fully connected to power supply and control unit 52. This ensures the nebulizer is properly primed before connecting to power source 52.

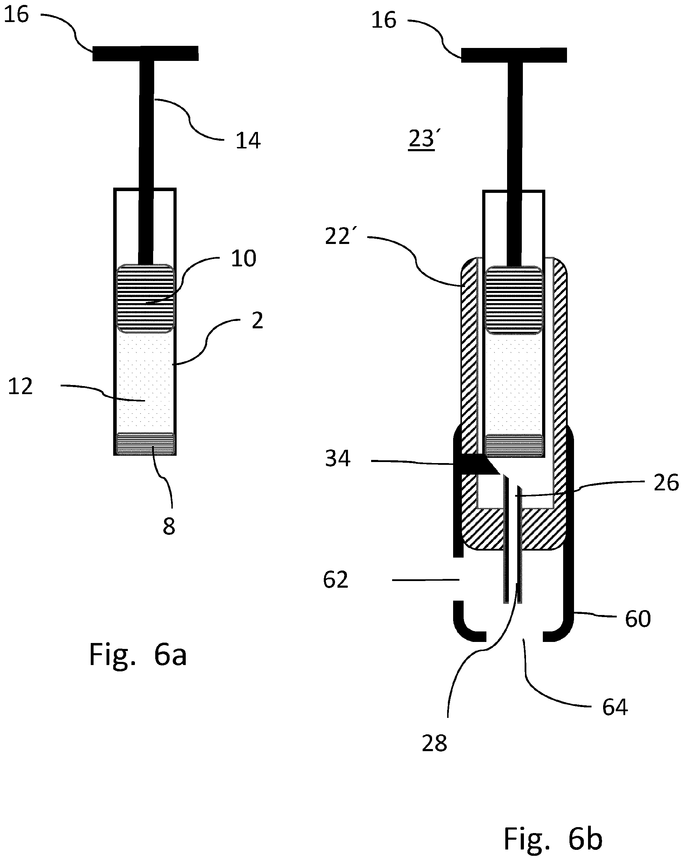

Fig. 6 shows another embodiment of the technology. In this embodiment, the housing 22' in which the cartridge 1 is provided, at first is not positioned in the nebulizer body 24 yet but is intended to be provided independently for later attachment to nebulizer body 24. For reference and comparison, Fig. 6a again shows the cartridge 1 and plunger 14. In the embodiment shown in Fig. 6b, cartridge housing 22' equipped with septum needle 26 has a nozzle cap 60 covering nozzle 28. Cap 60 has openings 62 and 64. Cap 60 is rotationally fixed to housing 22' and therefore is prevented from rotating on housing 22'. In this alternative, inventive embodiment the cartridge 1 is supplied to the user only assembled with the housing 22' and not also the nebulizer body 24. In this embodiment, in an aspect of the technology the cartridge 1 is prevented from moving axially downwards in housing 22' by lock 34 because locking pin 36 is prevented from moving out sideways by cap 60. This prevents the user inadvertently activating the device. The assembly shown in Figure 6b may be terminally sterilized in a blister. In total, the assembly shown in Fig. 6b is also considered to be a drug supply unit 23' in accordance with the technology, of a type that can be marketed or provided to the user as a separate, integer entity.

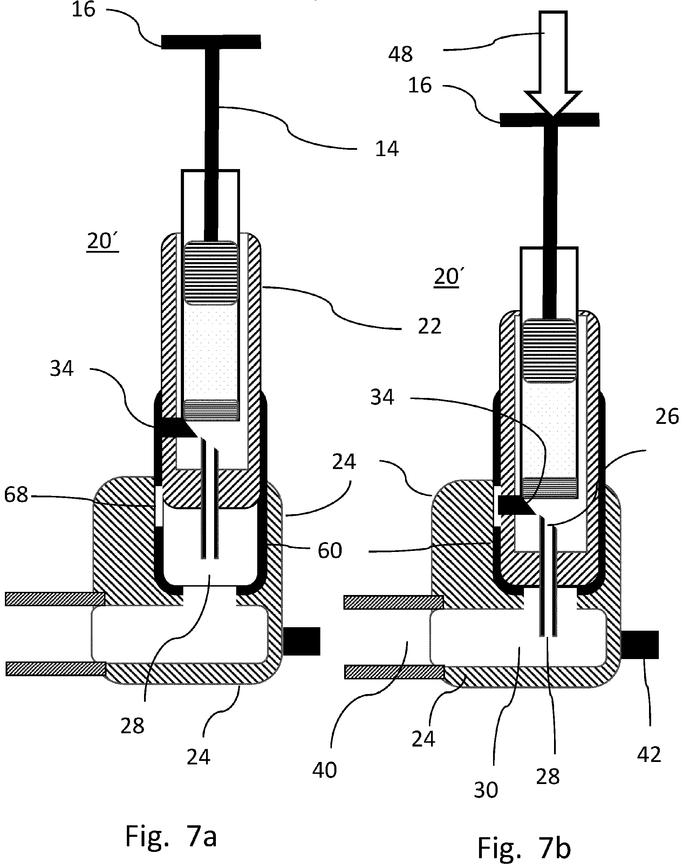

Fig. 7 shows the use of the cartridge 1 of Fig. 6 in a nebulizer system 20'. In Figure 7a the assembly of cartridge 1 and housing 22' are inserted into the nebulizer body 24. In Figure 7b the plunger 14 has been pressed by the user and in consequence cartridge 1 has moved downwards in receiving socket 66 in nebulizer body 24 together with housing 22'. Housing 22' consequently has moved axially relative to cap 60 allowing the nozzle 28 to enter nebulizer chamber 30 while at the same time aligning a release opening 68 of cap 60 with locking pin 36. According to an aspect of the present technology, the assembly is now ready for final unlocking of lock 34, which again may be effected by a twisting or rotating motion of housing 22' around its longitudinal direction until release opening 68 together with locking pin 36 rotationally overlap with corresponding recess 44 or cavity in nebulizer body 24. As in previous embodiment, recess 44 is designed such that locking pin 26 can slide freely therein such that in this orientational direction it is free to move radially outward and thereby no longer impedes cartridge 1 from being moved forward in housing 22' in the direction towards septum needle 26.

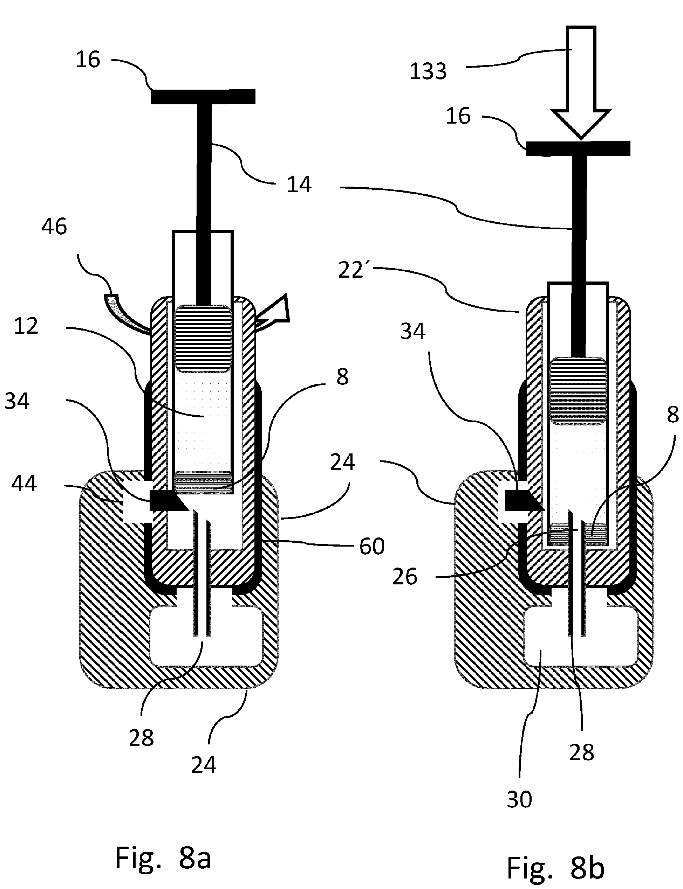

For illustration, Fig. 8a shows that housing 22' has rotated relative to nebulizer chamber 24 aligning release opening 68 and locking pin 34 with cavity 44. In Figure 8b the plunger 14 has been pressed by the user as shown by arrow 70 forcing septum needle 26 to pierce septum 8 ready for delivering contents 12 to nebulizer chamber 30 by further pressing plunger 14 as shown by arrow 72. In this embodiment, the position of lock 34 and cavity 44 may be reversed. More than one lock 34 may be used.

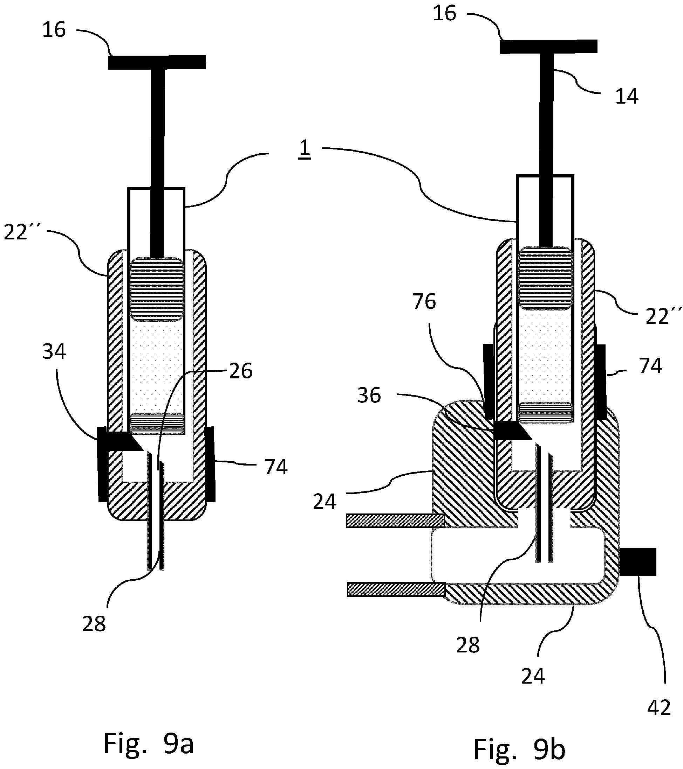

In yet another embodiment, shown in Fig. 9, instead of cap 60, a sleeve 74 slidingly positioned outside on housing 22" and containing release opening 68 may be provided. In this embodiment, no front protection is provided for nozzle 28. In this case the sleeve 74 may engage with a shoulder 76 in the nebulizer body 24. Alternatively, the sleeve 74 may be a short sleeve 74 as shown in Figs. 9a and 9b.

In the embodiment shown in Fig, 9a, sleeve 74 covers the locking pin 36 and therefore lock 34. Sleeve 74 is prevented from rotating on housing 22". In this embodiment the cartridge 1 also is supplied to the user only assembled with the housing 22" and not also the nebulizer body 24. In this embodiment, the cartridge 1 is prevented from moving axially downwards in housing 22" by lock 34 because locking pin 36 is prevented from moving out sideways by sleeve 74. This prevents the user inadvertently activating the device. In Figure 9b the syringe-type assembly of cartridge 1 and housing 22" are shown assembled into the nebulizer body 24. After pushing housing 22" into corresponding receiving socket 66 in nebulizer body 24, sleeve 74 has moved upwards by engaging with shoulder 76 in nebulizer body 24, thereby releasing locking pin 36 from coverage by sleeve 74. In this position, locking pin 36 now is prevented from moving out sideways by surrounding nebulizer body 24. From there, housing 22" may be rotated in nebulizer body 24 to bring locking pin 36 in overlap with cavity 44 such that locking pin 36 now is free to move out sideways. Fig. 9b shows the assembly before such final rotation to fully release lock 34.

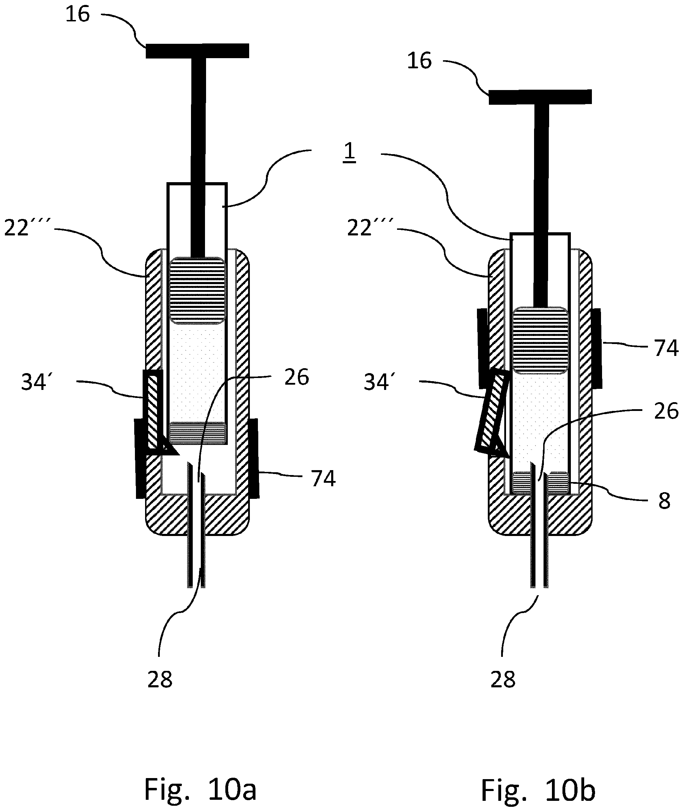

An alternative lock is shown in Fig. 10. Lock 34' is formed as part of housing 22‴. In Fig. 10a it is prevented from bending outwards by lock ring 80. In Fig. 10b lock ring 80 has been moved upwards freeing the lock 34' and allowing cartridge 1 to force lock 34' out of the way and allowing cartridge 1 to be pushed forward so that cartridge septum 8 is pierced by needle 26 ready for contents delivery into nebulizer chamber 30.

It is preferable that when the syringe-type assembly is rotated relative to the nebulizer body 24 that the rotation is limited by a stop to prevent over rotation.

The septum needle 8 may be any hypodermic type or other delivery needle. In a preferred embodiment the gauge of the needle 26 is as small as possible or with the largest diameter to minimize resistance when the contents are injected into the nebulizer. It is preferably that the gauge is between 16G and 26G.

In all the embodiments above there may be more than one lock 34, 34'.

The cartridge 1 may be filled with or without an air or gas bubble.

### List of reference numerals

- 1: Cartridge
- 2: barrel
- 4, 6: end
- 8: septum
- 10: stopper
- 12: drug formulation solution
- 14: plunger rod
- 16: finger pad
- 20,20': nebulization system
- 22,22': housing
- 23,23': drug supply unit
- 24: nebulizer body
- 26: septum needle
- 28: nozzle
- 30: nebulizer chamber
- 32: nebulization element
- 34: lock
- 36: locking wedge
- 38: distal end
- 40: mouth piece
- 42: electrical connector
- 44: recess
- 46, 48: arrow
- 50: inhalation device
- 52: power supply unit
- 54: connector
- 60: nozzle cap
- 62,64: openings
- 66: socket
- 68: release opening
- 70,72: arrow
- 74: sleeve
- 76: shoulder
- 80: lock ring

## Claims

1. Nebulization system (20, 20') comprising a drug supply unit (23, 23') for an inhalation device (50), said drug supply unit (23,23') comprising a cartridge (1) containing a drug formulation solution (12), with a housing (22, 22', 22", 22‴), in which the cartridge (1) is arranged so as to be displaceable in a longitudinal direction, the housing (22, 22', 22", 22‴) having a septum needle (26) in an end region, which, in an active position of the cartridge (1), pierces a septum (8) arranged at the distal end (6) of the cartridge (1) when the cartridge (1) is completely displaced in the longitudinal direction towards the end region, and having a releasable lock (34) which fixes the cartridge (1) in a passive position within the housing (22, 22', 22", 22‴) in such a way that the septum needle (26) is held at a distance from the septum (8), and further comprising a nebulizer body (24) with a nebulization chamber (30), wherein said septum needle (26) is in fluid communication with a nozzle (28) extending into said nebulization chamber (30), **characterized in that** unlocking of lock (34) is effected by a twisting or rotating motion of the housing (22, 22', 22", 22‴) around its longitudinal direction relative to the nebulizer body (24).

2. Nebulization system (20, 20') according to claim 1 in which said lock (34) comprises a locking pin (36) which in a locking position protrudes into the interior of the housing (22, 22', 22", 22‴).

3. Nebulization system (20, 20') according to claim 2 in which said locking pin (36) in an unlocked mode is allowed to move radially outward from the interior of the housing (22, 22', 22", 22‴).

4. Inhalation device (50) comprising a nebulization system (20, 20') according to any one of claims 1 to 3, connected to a power supply and control unit (52) by an electric connection system, wherein said electric connection system comprises a number of paired connector elements that are individualized with respect to the drug to be delivered.

## Patentansprüche

1. Vernebelungssystem (20, 20'), das eine Arzneimittelzuführeinheit (23, 23') für eine Inhalationsvorrichtung (50) umfasst, wobei die Arzneimittelzuführeinheit (23, 23') eine Kartusche (1) umfasst, die eine Arzneimittel-Formulierungslösung (12) enthält, mit einem Gehäuse (22, 22', 22", 22‴), in dem die Kartusche (1) so angeordnet ist, dass sie in einer Längsrichtung verschiebbar ist, wobei das Gehäuse (22, 22', 22", 22‴) in einem Endbereich eine Septumkanüle (26) aufweist, die, in einer aktiven Position der Kartusche (1), ein Septum (8), das an dem distalen Ende (6) der Kartusche (1) angeordnet ist, durchsticht, wenn die Kartusche (1) vollständig in der Längsrichtung hin zu dem Endbereich verschoben wird, und eine lösbare Arretierung (34) aufweist, welche die Kartusche (1) auf eine solche Weise in einer passiven Stellung innerhalb des Gehäuses (22, 22', 22'', 22‴) fixiert, dass die Septumkanüle (26) in einem Abstand von dem Septum (8) gehalten wird, und das ferner einen Verneblerkörper (24) mit einer Vernebelungskammer (30) umfasst, wobei die Septumkanüle (26) in Fluidverbindung mit einer Düse (28) steht, die sich in die Vernebelungskammer (30) erstreckt,
**dadurch gekennzeichnet, dass**
ein Entarretieren der Arretierung (34) durch eine verdrehende oder drehende Bewegung des Gehäuses (22, 22', 22", 22‴) um seine Längsrichtung im Verhältnis zu dem Verneblerkörper (24) bewirkt wird.

2. Vernebelungssystem (20, 20') nach Anspruch 1, wobei die Arretierung (34) einen Arretierungsstift (36) umfasst, der in einer Arretierungsstellung in das Innere des Gehäuses (22, 22', 22", 22‴) vorspringt.

3. Vernebelungssystem (20, 20') nach Anspruch 2, wobei dem Arretierungsstift (36) in einem entarretierten Modus ermöglicht wird, sich von dem Inneren des Gehäuses (22, 22', 22'', 22‴) in Radialrichtung nach außen zu bewegen.

4. Inhalationsvorrichtung (50), die ein Vernebelungssystem (20, 20') nach einem der Ansprüche 1 bis 3 umfasst, das durch ein elektrisches Verbindungssystem mit einer Energieversorgungs- und Steuerungseinheit (52) verbunden ist, wobei das elektrische Verbindungssystem eine Anzahl von paarweisen Verbinderelementen umfasst, die in Bezug auf das Arzneimittel, das verabreicht werden soll, individualisiert sind.

## Revendications

1. Système de nébulisation (20, 20') comprenant une unité d'alimentation en médicament (23, 23') pour un dispositif d'inhalation (50), ladite unité d'alimentation en médicament (23, 23') comprenant une cartouche (1) contenant une solution de formulation de médicament (12), avec un boîtier (22, 22', 22", 22‴) dans lequel la cartouche (1) est disposée de manière à être déplaçable dans le sens longitudinal, le boîtier (22, 22', 22'', 22‴) présentant dans une zone d'extrémité une aiguille à septum (26) qui, dans une position active de la cartouche (1), perce un septum (8) disposé à l'extrémité distale (6) de la cartouche (1) lorsque la cartouche (1) est complètement déplacée dans le sens longitudinal vers la zone d'extrémité, et comprenant un verrou libérable (34) qui fixe la cartouche (1) dans une position passive à l'intérieur du boîtier (22, 22', 22'', 22‴) de telle sorte que l'aiguille de septum (26) soit maintenue à une certaine distance du septum (8), et comprenant en outre un corps de nébuliseur (24) avec une chambre de nébulisation (30), ladite aiguille de septum (26) étant en communication fluidique avec une buse (28) s'étendant dans ladite chambre de nébulisation (30), **caractérisé en ce que**
le déverrouillage du verrou (34) s'effectue par un mouvement de torsion ou de rotation du boîtier (22, 22', 22'', 22‴) autour de sa direction longitudinale par rapport au corps de nébuliseur (24).

2. Système de nébulisation (20, 20') selon la revendication 1, dans lequel ledit verrou (34) comprend une broche de verrouillage (36) qui, dans une position de verrouillage, fait saillie à l'intérieur du boîtier (22, 22', 22‴, 22‴).

3. Système de nébulisation (20, 20') selon la revendication 2, dans lequel ladite broche de verrouillage (36), dans un mode déverrouillé, peut se déplacer radialement vers l'extérieur depuis l'intérieur du boîtier (22, 22', 22", 22‴).

4. Dispositif d'inhalation (50) comprenant un système de nébulisation (20, 20') selon l'une quelconque des revendications 1 à 3, connecté à une unité d'alimentation et de commande (52) par un système de connexion électrique, ledit système de connexion électrique comprenant un certain nombre d'éléments de connexion appariés qui sont individualisés par rapport au médicament à administrer.
